# EUROPÄISCHE PATENTANMELDUNG

(11) **EP 1 174 491 A2**
(43) Veröffentlichungstag der Anmeldung: **23.01.2002**
(21) Anmeldenummer: 01250274.6
(22) Anmeldetag: 20.07.2001
(51) Int. Cl.: C11D 3/39

(54) **Bleich- und Oxidationsmittel und ihre Verwendung**

(30) Priorität: 21.07.2000 DE 10037162
(71) Anmelder: Hahn, Ekkehardt, 14055 Berlin (DE); Dittler-Klingemann, Andreas, 15732 Eichwalde (DE)
(72) Erfinder: Hahn, Ekkehardt, 14055 Berlin (DE); Dittler-Klingemann, Andreas, 15732 Eichwalde (DE)
(74) Vertreter: Wehlan, Helmut, Dr.

(57) **Zusammenfassung**

Die vorliegende Erfindung betrifft Bleich- und Oxidationsmittel, die ein- oder mehrkernige Metall-Komplexe mit unsymmetrisch substituierten tripodalen Liganden enthalten. Unsymmetrisch bedeutet, dass sich die Substituenten am zentralen Ligandenatom des Tripods durch die Länge der Alkylengruppen zwischen dem zentralen Ligandenatom und den direkt koordinierenden Donoratomen und/oder durch unterschiedliche Donoratome unterscheiden. Die Erfindung betrifft weiterhin die Verwendung dieser Metallkomplexe als Bleich- und Oxydationskatalysatoren in Wasch- und Reinigungsmitteln, Wasch- und Reinigungsmittel-Formulierungen, zum Bleichen von Textilien, Papier, verschmutzten Substraten oder zum Färben bzw. Bleichen von Haaren sowie zur Aktivierung von Luftsauerstoff.

## Beschreibung

Die vorliegende Erfindung betrifft Bleich- und Oxidationsmittel, die ein- oder mehrkernige Metall-Komplexe mit tripodalen Liganden enthalten. Die Erfindung betrifft weiterhin die Verwendung dieser Metallkomplexe als Bleich- und Oxydationskatalysatoren. Mögliche Anwendungsgebiete sind Wasch-, Haarfärbe-, Reinigungs- und Bleichmittel.

Peroxidbleichmittel, insbesondere Wasserstoffperoxid und feste Persauerstoffverbindungen, die unter Freisetzung von Wasserstoffperoxid reagieren, wie Natriumperborat und Natriumcarbonat-perhydrat, werden seit geraumer Zeit als Oxidationsmittel zu Desinfektions- und Bleichzwecken, insbesondere für Textilwäsche und in einer Vielzahl industrieller Prozesse sowie in haushaltsüblichen Wasch- und Reinigungsmitteln verwendet. Die Oxidationswirkung dieser Substanzen ist in verdünnter Lösung stark temperaturabhängig. Beispielsweise wird mit H₂O₂ unter alkalischen Bedingungen erst oberhalb 80 °C eine ausreichend schnelle Bleiche verschmutzter Textilien erreicht. Auch bei niedrigeren Temperaturen kann die Oxidationswirkung von Persauerstoffverbindungen durch Zusatz sogenannter Bleichaktivatoren aus der Stoffklasse der N- oder O-Acylverbindungen verbessert werden. Durch den Zusatz dieser Aktivatoren kann die Bleichwirkung wässriger Peroxidlösungen derart gesteigert werden, dass bereits bei Temperaturen um 60 °C im wesentlichen gleiche Wirkungen wie mit Peroxidlösungen allein bei 95 °C beobachtet werden. Die Bleichaktivatoren reagieren mit dem Peroxosauerstoff-haltigen Bleichmittel und bilden Peroxosäuren, die ihrerseits die Verschmutzungen am Reinigungsgut oxidieren. Eine solche Reaktion ist nicht katalytisch, d.h. wenn der Aktivator reagiert hat, ist er verbraucht und kann nicht nochmals in Reaktion treten. Daher ist es nötig, relativ hohe Mengen an Bleichaktivator zu verwenden, was erhebliche Kosten verursacht. Diese Eigenschaft wie auch die relativ hohen Bleichtemperaturen um 60 °C sind hinsichtlich der ökonomischen wie auch praktischen Prozessführung von erheblichem Nachteil.

Hinsichtlich der Entwicklung energie- und ressourcensparender Wasch- und Bleichverfahren kommt Anwendungstemperaturen deutlich unter 60 °C, insbesondere unter 45 °C bis hin zu Kaltwassertemperaturen sowie der Aktivierung von Luftsauerstoff für Bleichanwendungen eine besondere Bedeutung zu.

Bei niedrigen Temperaturen lässt die Wirkung der bisher beschriebenen Aktivatorverbindungen in der Regel erheblich nach. Versuche zur Entwicklung von Aktivatoren für diesen Temperaturbereich oder zur Aktivierung von Luftsauerstoff für Bleichzwecke sind bisher ohne überzeugenden Erfolg geblieben.

Einen anderen Ansatz stellt die Verwendung von Übergangsmetallsalzen oder - komplexen als sogenannte Bleichkatalysatoren, wie beispielsweise in den europäischen Patentanmeldungen EP 0 392 592, EP 0 443 651, EP 0 458 397, EP 0 544 490 oder EP 0 549 271 vorgeschlagen, dar. In der Patentschrift US 728455 wird zum Beispiel Mangan(III)gluconat als Bleichkatalysator beschrieben. In EP 458 397 werden auf Triazacyclononan basierende Mangankomplexe vorgestellt. In den Patentschriften WO 95/30681 und DE 197 55 493 werden Cu-, Mn-, Co- und Zn-Komplexe, teils mit mehrzähnigen - unter Einschluss symmetrischer tripodaler - Liganden als Bleichkatalysatoren beschrieben. In den internationalen Patentschriften WO 95/34628, WO 97/18035 und WO 00/27976 werden Komplexe, teils mit mehrzähnigen pyridylhaltigen Liganden, als Bleichkatalysatoren beschrieben. In der Schrift WO 95/30681 werden Metallkomplexe mit Polyaminliganden präsentiert.

Diese Bleichkatalysatoren zeigen teilweise eine hohe Aktivität. Allerdings besteht, gerade wegen der hohen Reaktivität des wahrscheinlich aus der Persauerstoffverbindung und dem Bleichkatalysator gebildeten Intermediates, die Gefahr der Farbveränderung und der oxidativen Textilschädigung. Weiterhin sind die oben angesprochenen Komplexe in ihrer Wirksamkeit, Stabilität und Verträglichkeit als Bleichkatalysatoren nicht voll befriedigend. Insbesondere die Aktivierung von Peroxoverbindungen bei niedrigen Temperaturen unter 45 °C gelingt nicht.

In den Dokumenten WO 01/05925 und DE 44 16 438 werden Komplexe mit tripodalen Liganden und deren Verwendung als Katalysatoren beschrieben. Diese tripodalen Liganden enthalten als Donoratome und als zentrales Gerüstatom ausschließlich N-Atome. In der Schrift WO 01/05925 befinden sich zwischen den Stickstoffatomen Ethylenbrücken. Im Dokument DE 44 16 438 wird beschrieben, dass sich zwischen den Donoratomen und dem zentralen Gerüstatom sich C₂ bis C₄-Alkylengruppen befinden, jedoch sind diese Alkylengruppen jeweils symmetrisch angeordnet, d.h., es sind entweder C₂ oder C₃ oder C₄-Gruppen vorhanden. In dieser Offenlegungsschrift wird zwar über die Möglichkeit berichtet, andere Koordinationsmoleküle als Stickstoff einzusetzen, das betrifft aber nicht die dort dargestellten tripodalen Liganden.

Die zum Bleichen menschlicher Haare verwendeten oxidierenden Zubereitungen bestehen üblicherweise aus einer Mischung anorganischer Peroxosalze, Alkalisalzen, Ammoniumsalzen und die Viskosität regulierenden Stoffen. Beispiele für solche Rezepturen findet man in der Fachliteratur, wie bei K. Schrader, Grundlagen und Rezepturen der Kosmetika, 2. Aufl. 1989, Hütig Verlag, S. 815 bis 823, weiterhin in den Dokumenten EP 630 643 und DE 195 43 989. Auch hierin ist ein schonendes Verfahren unter Umgehung der potentiellen Haarschädigung durch stark oxidierende Bleichmittel noch nicht beschrieben worden.

Aufgabe der vorliegenden Erfindung war es daher, Bleichkatalysatoren mit verbesserter Wirksamkeit bereitzustellen, die die Mängel der im Stand der Technik beschriebenen Systeme nicht mehr aufweisen.

Die Aufgabe wurde durch Bleich- und Oxidationsmittel gelöst, die Metallkomplexe mit einem oder mit mehreren unsymmetrisch substituierten tripodalen Liganden enthalten. Unsymmetrisch bedeutet, dass sich die Substituenten am zentralen Ligandenatom des Tripods durch die Länge der Alkylengruppen zwischen dem zentralen Ligandenatom und den direkt koordinierenden Donoratomen und/oder durch unterschiedliche Donoratome unterscheiden.

Die erfindungsgemäßen Bleich- und Oxidationsmittel können folgende Substituenten am zentralen Ligandenatom des Tripods enthalten:
a) mindestens zwei unterschiedliche Alkylengruppen zwischen den Donoratomen, aber gleiche Donorgruppen
   oder
b) gleiche Alkylengruppen zwischen den Donoratomen, aber Donorgruppen mit mindestens zwei unterschiedlichen Donoratomen
   oder
c) mindestens zwei unterschiedliche Alkylengruppen zwischen den Donoratomen und unterschiedliche Donorgruppen besitzen.

Überraschenderweise hat sich herausgestellt, dass Übergangsmetallkomplexe unsymmetrischer tripodaler Liganden erfindungsgemäß im Zusammenwirken mit organischen und anorganischen Peroxosauerstoffverbindungen oder auch unter Aktivierung atmosphärischen Sauerstoffs zu einer deutlichen Steigerung der Bleichwirkung gegenüber gefärbten Anschmutzungen, die sich an Textilien oder auf harten Oberflächen befinden bzw. zur Bleichung menschlicher oder tierischer Haare bei Temperaturen unter 60 °C beitragen.

Die erfindungsgemäßen Bleich- und Oxidationsmittel enthalten ein- oder mehrkernige Komplexe, vorzugsweise der Übergangsmetalle Cobalt, Kupfer, Nickel, Mangan, Eisen, Zink oder Vanadium, die einen oder mehrere Liganden aus folgenden Klassen besitzen:
a) unsymmetrische tripodale Liganden mit zentralem Element der 5. Hauptgruppe der allgemeinen Formeln I : 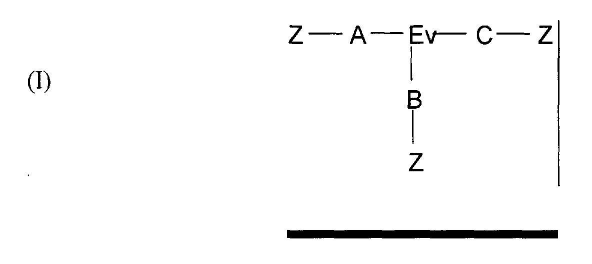 in der Eᵥ ein Element der 5. Hauptgruppe, A, B und C eine C₂- bis C₄-Alkylengruppe bezeichnen mit A ≠ B oder A ≠ B ≠ C; Z, für -NR¹R², -SR³ und -OR⁴ steht, wobei R¹ bis R⁴ für Wasserstoff, Alkyl, Aryl und substituiertem Aryl stehen.
b) tripodale Liganden mit zentralem Stickstoffatom der allgemeinen Formeln II: 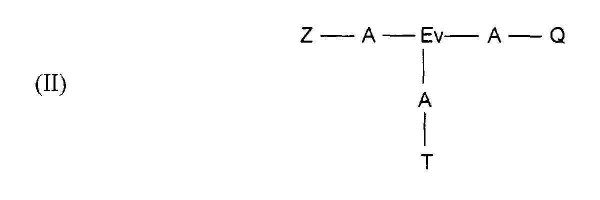 in der Eᵥ ein Element der 5. Hauptgruppe, A eine C₂- bis C₄-Alkylengruppe bezeichnet, wobei Z, Q und T für -NR¹R², -SR³ und -OR⁴ stehen, mit Z ≠ Q oder Z ≠ Q ≠ T, wobei R¹ bis R⁴ für Wasserstoff, Alkyl, Aryl und substituiertem Aryl stehen und sich Z, Q und T mindestens in einem Donoratom unterscheiden, wobei R¹ bis R² für Wasserstoff, Aryl und substituiertes Aryl, insbesondere mono oder bis Amino-, Hydroxy- und Mercaptoaryl stehen.
c) tripodale Liganden mit zentralem Stickstoffatom der allgemeinen Formeln III 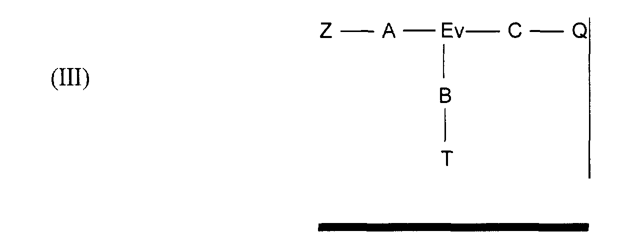 in der Eᵥ ein Element der 5. Hauptgruppe, A, B und C eine C₂- bis C₄-Alkylengruppe bezeichnen mit A ≠ B oder A ≠ B ≠ C, Z, Q und T unabhängig voneinander mit Z ≠ Q oder Z ≠ Q ≠ T, wobei Z, Q und T für -NR¹R², -SR³ und -OR⁴ stehen, wobei R¹ bis R⁴ für Wasserstoff, Alkyl, Aryl und substituiertem Aryl stehen und sich Z, Q und T mindestens in einem Donoratom unterscheiden.

Die erfindungsgemäßen Bleich- und Oxidationsmittel enthalten ein- oder mehrkernige Metall-Komplexe der allgemeinen Formel IV :

(IV) [LₐM_{b}X_{c}]^{d} Y,

in der M für ein Übergangsmetall Oxidationsstufe I, II, III, IV, V und/oder VI steht, X eine Koordinations- oder Brückengruppe darstellt, Y ein Gegenion in der entsprechenden stöchiometrischen Menge zum Ausgleich einer vorhandenen Ladung d bezeichnet, wobei d als Ladung des Metall-Komplexes positiv, null oder negativ sein kann, a und b unabhängig voneinander ganze Zahlen von 1 bis 8 bedeuten, c eine ganze Zahl von 0 bis 32 bezeichnet und L für ein Element der 5. Hauptgruppe steht.

Die beschriebenen Metall-Komplexe eignen sich erfindungsgemäß als Bleich- und Oxidationskatalysatoren, z.B. in Verbindung mit organischen und anorganischen Persauerstoffverbindungen, insbesondere in Wasch- und Reinigungsmitteln und bei der Textil- und Papierbleiche. Sie können in Verfahren zum Bleichen von verschmutzten Substraten Verwendung finden und Bestandteile in Wasch- und Reinigungsmittel-Formulierungen sein. Außerdem können sie zum Färben bzw. Bleichen von Haaren verwendet werden.

Die genannten Übergangsmetalle in den erfindungsgemäß verwendeten Bleichkatalysatoren liegen vorzugsweise in den Oxidationsstufen +2, +3, +4 oder +5 vor. Komplexe mit unterschiedlichen Metallatomen bzw. gleichen Metallatomen in gleicher oder unterschiedlichen Metallatomen bzw. gleichen Metallatomen in gleicher oder unterschiedlicher Oxidationsstufe sind möglich.

Außer den Liganden der allgemeinen Formeln I-III können die Übergangsmetallkomplexe noch weitere, in der Regel einfacher aufgebaute Liganden, insbesondere Neutral- beziehungsweise ein- oder mehrwertige Anionenliganden, tragen. In Frage kommen beispielsweise Cl⁻, Br⁻, I⁻, F⁻, SCN⁻, I₃⁻, OH⁻, O₂²⁻, O²⁻, O₂⁻, OOH⁻, H₂O, SH⁻, CN⁻, OCN⁻, HCO₃⁻, CO₃²⁻, S₄²⁻, S²⁻, SO, N₃⁻, N³⁻, NH₃, N(R⁵)₃, N(R⁵)₂⁻, R⁵O⁻, R⁵COO⁻, (R⁵)₃C-CO-CH-C(O⁻)-(R⁵)₃, R⁵SO₃⁻ und R⁵SO₄-, wobei R⁵ jeweils Wasserstoff, C1- bis C8-Alkyl, C7- bis C15-Aralkyl oder C6-bis C18-Aryl bezeichnet.

Die Übergangsmetallkomplexe können als negativ oder positiv geladene Komplexe wie auch als Neutralkomplexe auftreten. Geladene Komplexe enthalten Gegenionen. Bei positiv geladenen Komplexen werden diese bevorzugt ausgewählt aus Cl⁻, Br⁻, I⁻, F⁻, NO₃⁻, ClO₄⁻, SCN⁻, PF₆⁻, R⁵SO₄⁻, R⁵COO⁻, CF₃COO⁻, R⁵SO₃⁻, BF₄⁻, B(Ph)₄⁻, SO₄²⁻ and SO₃²⁻, - bei negativem d: Li⁺, Na⁺, K⁺, Mg²⁺, Ca²⁺, Sr²⁺, Ba²⁺, NH₄⁺, R⁵NH₃⁺, (R⁵)₂NH₂⁺, (R⁵)₃NH⁺ und (R⁵)₄N⁺, wobei R⁵ die oben genannte Bedeutung hat.

Die erfindungsgemäßen Bleich- und Oxidationsmittel zeigen eine Reihe von Eigenschaften, die wesentliche Nachteile der bereits beschriebenen Bleichkatalysatoren überwinden. Durch den Einsatz von topologisch unsymmetrischen tripodalen Liganden lassen sich die Eigenschaften der von diesen Liganden komplexierten Metallatome über einen weiten Bereich gezielt variieren. Durch Variation der Zahl der fünf- und sechgliedrigen Chelatringe lassen sich das Ligandenfeld um das Metallzentrum sowie davon abgeleitete Eigenschaften verändern. Beispielsweise variiert die Stabilität von Kupferkomplexen mit tripodalen Tetraaminen mit der Zahl der Propyl-Ligandenarme (Andreas M. Dittler-Klingemann and F. Ekkehardt Hahn: "Trigonal-Bipyamidal Copper(II) Complexes with Symmetric and Unsymmetric Tripodal Tetramine Ligands", Inorg. Chem.1996, 35, 1996; Andreas M. Dittler-Klingemann, Chris Orvig, F. Ekkehardt Hahn, Florian Thaler, Colin D. Hubbard, Rudi van Eldik, Siegfried Schindler and Istvan Fabian: "Geometric Factors in the Structural and thermodynamic Properties of Copper(II)Complexes with tripodal Tetramines", Inorg. Chem. 1996, 35, 7798). Kupfer(I)-Komplexe dieser Liganden sind teilweise in der Lage, direkt atmosphärischen Sauerstoff zu aktivieren und für Oxidationsreaktionen bereitzustellen. Ein weiteres Beispiel für die Aktivierung von Luftsauerstoff für Oxidationsprozesse wurde erfindungsgemäß mit dem Liganden N(CH₂CH₂SH)(CH₂CH₂NH₂)(CH₂CH₂CH₂OH) gefunden. Dieser topologisch wie auch von der Art der Donorfunktionen unsymmetrische tripodale Ligand ist ebenfalls in der Lage, im Metallkomplex Luftsauerstoff für Oxidationsreaktionen zu aktivieren.

Besonders hervorzuheben sind hier Textilwaschmittel in Form von Pulverwaschmitteln oder als flüssige Formulierungen und Geschirr-Reinigungsmittel. Ein Vorteil der erfindungsgemäßen Bleichkatalysatoren ist dabei ihre Stabilität gegenüber Hydrolyse und Oxidation. Die Metall-Komplexe IV selbst besitzen die katalytische Aktivität und sind insbesondere in einer Vielzahl von Textilwaschmittelformulierungen hochwirksam.

Als weitere anwendungstechnische Vorteile, insbesondere in Textilwaschmittelformulierungen, sind ihre universelle Wirksamkeit bei der Entfernung aller Arten von Verschmutzungen, sowohl hydrophiler als auch hydrophober Natur, und ihre Verträglichkeit mit den üblichen Waschmittel-Enzymen wie Proteasen, Cellulasen, Lipasen, Amylasen oder Oxidasen zu nennen.

Gegenstand der vorliegenden Erfindung ist demgemäss auch ein Verfahren zum Bleichen von verschmutzten Substraten, welches dadurch gekennzeichnet ist, dass man das verschmutzte Substrat in wässriger Bleichflotte mit Peroxyverbindungen und einer wirksamen Menge eines oder mehrerer ein- oder mehrkerniger Metall-Komplexe I als Bleichkatalysatoren in Kontakt bringt, um miteinander zu wechselwirken und eine Reinigungswirkung auf dem Substrat zu erzielen.

Gegenstand der vorliegenden Erfindung sind weiterhin Wasch- und Reinigungsmittel-Formulierungen, enthaltend neben den üblichen Bestandteilen übliche Mengen an Peroxyverbindungen und eine wirksame Menge eines oder mehrerer ein- oder mehrkerniger Metall-Komplexe I als Bleichkatalysatoren.

Peroxyverbindungen, die zusammen mit den erfindungsgemäßen Bleichkatalysatoren verwendet werden können, umfassen Wasserstoffperoxid, Wasserstoffperoxid freisetzende Verbindungen, Wasserstoffperoxid erzeugende Systeme, Peroxysäuren und ihre Salze und Peroxysäure-Vorstufen sowie Mischungen hieraus.

Als Wasserstoffperoxid-Quellen sind beispielsweise Alkalimetallperoxide, Harnstoff-H₂O₂-Komplexe, Alkalimetallperborate, -percarbonate, -perphosphate und -persulfate bekannt. Von besonderer Bedeutung sind Natriumperborat-Monohydrat und -Tetrahydrat. Die genannten Bleichmittel können in Kombination mit Peroxysäure-Vorstufen eingesetzt werden.

Beispiele für Peroxysäure-Vorstufen mit quaternären Ammonium-Strukturen sind 2-(N,N,N Triethylammonium)ethyl-4-sulfophenylcarbonat, N-Octyl-N,N-dimethyl-N-10-carbophenoxy-decylammoniumchlorid, 3-(N,N,N-Trimethylammonium)propyl-Natrium-4-sulfophenyl-carb-oxylat und N,N,N-Trimethylammonium-toluyloxybenzolsulfonat.

Bevorzugte Klassen von Bleichmittel-Vorstufen, d. h. Peroxysäure-Vorstufen, sind neben den oben genannten quaternären Ammoniumsalzen Ester einschließlich der Acylphenolsulfonate und der Acylalkylphenolsulfonate sowie Acylamide.

Von besonderem Interesse sind hierbei die in der Praxis gern eingesetzten, oft auch als Bleichaktivatoren bezeichneten Verbindungen Natrium-4-benzoyloxybenzolsulfonat, N,N,N min, N-min-Tetraacetylethylendiamin (TAED), Natrium-1-methyl-2-benzoyloxy-benzol-4-sulfonat, Natrium-4-methyl-3-benzoyloxy-benzoat, Natriumnonanoyloxybenzolsulfonat, Natrium-3,5,5-trimethylhexanoyloxybenzolsulfonat, 2-Phenyl-benz-(4H)1,3-oxazin-4-on, Glucosepentaacetat und Tetraacetylxylose.

Auch aliphatische oder aromatische Mono- oder Dipercarbonsäuren eignen sich als Peroxyverbindungen. Beispiele hierfür sind Peroxy-alpha-naphthoesäure, Peroxylaurinsäure, Peroxystearinsäure, N,N-Phthaloylaminoperoxycapronsäure, 1,12-Diperoxydodecandisäure, 1,9-Diperoxyazelainsäure, Diperoxysebacinsäure, Diperoxyisophthalsäure, 2-Decyldiperoxybutan-1,4-disäure und 4,4,-Sulfonylbisperoxybenzoesäure.

Weiterhin eignen sich als Peroxyverbindungen anorganische Peroxysäure-Salze, z. B. Kaliummonopersulfat.

Die erfindungsgemäßen Wasch- und Reinigungsmittel-Formulierungen enthalten üblicherweise 1 bis 30 Gew.-%, insbesondere 2 bis 25 Gew.-% an Peroxyverbindungen.

Die erfindungsgemäßen Wasch- und Reinigungsmittel-Formulierungen enthalten in der Regel weiterhin die hierfür üblichen Bestandteile in den üblichen Mengen, d. h. insbesondere oberflächenaktive Substanzen und Gerüst- oder Builder-Substanzen. Die erfindungsgemäßen Bleichkatalysatoren sind mit diesen Bestandteilen der Formulierungen sowie mit weiteren gegebenenfalls mit enthaltenen Hilfsmitteln weitgehend verträglich.

Bei anwendungstechnischen Tests zeigten marktübliche Kompaktwaschmittelformulierungen, die die erfindungsgemäßen Bleichkatalysatoren in den angegebenen Mengen enthielten, eine beträchtliche Erhöhung der Bleichwirkung, insbesondere bei mit Tee und Rotwein verschmutztem Textilgewebe.

Die Merkmale der Erfindung gehen außer aus den Ansprüchen auch aus der Beschreibung hervor, wobei die einzelnen Merkmale jeweils für sich allein oder zu mehreren in Form von Kombinationen vorteilhafte schutzfähige Ausführungen darstellen, für die mit dieser Schrift Schutz beantragt wird. Die Kombination besteht aus bekannten Elementen (Bleich- und Oxidationskatalysatoren sowie Metallkomplexen mit unsymmetrisch substituierten tripodalen Liganden), die sich gegenseitig beeinflussen und in ihrer neuen Gesamtwirkung einen Gebrauchsvorteil und den erstrebten Erfolg ergeben, der darin liegt, dass Bleich- und Oxidationskatalysatoren in verbesserter Qualität und mit größeren Variationsmöglichkeiten zur Verfügung gestellt werden.

Die folgenden Beispiele dienen der Verdeutlichung der Erfindung, ohne sie auf diese Beispiele zu beschränken.

### Ausführungsbeispiele

### Beispiel 1: Herstellung der Metallkomplexe und der Liganden

Die Synthesen der genannten Liganden L und die Herstellung der Metall-Komplexe I hieraus beruhen auf an sich bekannten Methoden und sind dem Fachmann geläufig. z.B. C. Ochs, F. E. Hahn, R. Fröhlich: "An Unsymmetrical Tripodal Ligand with an N₂SO Donor Set: Coordination Chemistry with Nickel(II) Ions and Aerial Oxidation to the Sulfinate Complex", *Chem. Eur*. *J.* 2000, *6*, 2193-2199; A. M. Dittler-Klingemann, F. E. Hahn, "Trigonal-Bipyramidal Copper(II) Complexes with Symmetric and Unsymmetric Tripodal Tetramine Ligands", *Inorg. Chem.* 1996, *35*, 1996-1999.

### Beispiel 2: Liganden L in ein- oder mehrkernigen Metall-Komplexen

2.1. N-(2-Aminoethyl)N,N-bis(3-aminopropyl)amin, H₂NCH₂CH₂N(CH₂CH₂CH₂NH₂)₂
¹H-NMR (CDCl₃, 250 Mz, δ, ppm): 2.76 (t, 6H, H₂NC*H*₂CR₂); 2.52 (t, 6H, NC*H*₂CH₂); 1.58 (q, 4R, CH₂C*H*₂CH₂); 1.28 (s, 6H, CH₂N*H*₂)
¹³C-NMR (CDCl₃, 62.90 Mz,δ, ppm):
56.39 (N*C*H₂CH₂NH₂); 51.3 (N*C*H₂CH₂CH₂); 39.63 (CH₂CH₂*C*H₂NH₂); 38.92 (CH₂*C*H₂NH₂); 30.23 (CH₂*C*H₂CH₂)

| CHN-Analyse: C₈H₂₂N₄ M = 174.29 g/mol | | | |
|---|---|---|---|
| Ber. | C55.13 | H12.72 | N32.15 |
| Gef | C 54.46 | H13.22 | N31.82 |

2.2. N,N-Bis(aminoethyl)N-(3-aminopropyl)amin, (H₂NCH₂CH₂)₂NCH₂CH₂CH₂NH₂
¹H-NMR (CDCl₃,250 Mz, δ, ppm):
2.50 (CH₂C*H*₂NH₂); 2.26 NC*H*₂CH₂); 1.40 (CH₂C*H*₂CH₂); 1.06 N*H*₂*)*
¹³C-NMR ( CDCl₃, 62.90 Mz, δ, ppm):
57.95 N*C*H₂CH₂); 50.87 (N*C*H₂CH₂CH₂); 40.53 (NCH₂CH₂NH₂); 39.39 (CH₂CH₂*C*H₂NH₂); 29.93 (CH₂*C*H₂CH₂)

| CHN-Analyse: C₇H₂₀N₄ M= 160.27g/mol | | | |
|---|---|---|---|
| Ber. | C 52.46 | H 12.58 | N 34.96 |
| Gef | C 52.93 | H 12.34 | N 34.02 |

2.3. 2-Hydroxy-N,N-bis(2-aminoethyl)-ethylamin, HOCH₂CH₂N(CH₂CH₂NH₂)₂
¹H-NMR (CDCl₃, 250 MHz, δ, ppm):
3.62 (t, 2H, -C*H*₂O); 2.79 (t, 4H, -C*H*₂NH₂); 2.63 (m, 6H, N^{t}CH₂-); 2.19 (s, br, 5H, N*H*₂ + OH)
¹³C-NMR (CDCl₃, 62.90 MHz, δ, ppm):
60.05(-*C*H₂O); 57.38 (N^{t}*C*H₂CH₂OH); 56.28 (N^{t}*C*H₂CH₂NH₂); 39.78 (-*C*H2NH2)
MS (EI, *m*/*z*, rel. Int.):
148 (0.21, [M]+); 130(1.42, [M-H₂O]+; 117(75.71); 74 (57.61); 44(100); 30(21.80)
IR (KBr, v, cm⁻¹) :
3347, 3272, 3175 [s, V(NH2) + v(OH)]; 2937, 2869 [s, v(C-R)]; 1655 [m, 8(NH2)];1295 [m, v(C-N)]; 1049 [s, v(C-O)]
2.4. 2-Amino-N,N-bis(2-hydroxyethyl)-ethylamin, H₂NCH₂CH₂N(CH₂CH₂OH)₂
¹H-NMR (CDCl₃, 400 MHz, δ, ppm):
3.61 (s, br, 4H,-O*H* + -N*H*₂); 3.41 (t, 4H, -C*H*₂O); 2.59 (**t**, 211, N^{t}C*H*₂CH₂NH₂); 2.43 (t + t, 6H, N^{t}C*H*₂CH₂OH + -C*H*₂NH₂)
¹³C-NMR (CDCl₃, 100 MHz, δ, ppm):
58.98 (-*C*H₂0); 57.77 (N^{t}*C*H₂CH₂OH); 56.42 (N^{t}*C*H₂CH₂NH₂); 39.13 (-*C*H₂NH₂)
MS (FAB, positive Ionen, *m*/*z*, rel. Int.):
149 (100, M+H]+]; 118(47.42); 88 (22.03); 74(19.99); 56(16.61); 44(46.81)

| CHN-Analyse: C₆H₁₆N₂0₂ M= 148.21 g/mol | | | |
|---|---|---|---|
| Ber. | C48.63 | H 10.88 | N18.90 |
| Gef | C47.90 | H 10.05 | N17.88 |

2.5. 3-Hydroxy-N-(2-aminoethyl)-N-(3-hydroxypropyl)-amin, HOCH₂CH₂CH₂N(CH₂CH₂CH2OH)(CH2CH2NH2)
1H-NMR (CDCl₃, 250 MHz, δ, ppm):
3.67 (s + t, 6H, -O*H* + -C*H*₂O); 2.80 (t, 2H, N^{t}*CH*₂CH₂NH₂); 2.58 (t, 4H, N^{t}*CH*₂CH₂CH₂OH); 2.50 (t, 2H, -*CH*₂NH₂), 1.70 (m +s,6H, CH₂*CH*₂CH₂ + -N*H*₂)
¹³C-NMR (CDCl₃, 62.90 MHz, δ, ppm):
61.27 (-*C*H₂0); 56.45 (N^{t}*C*H₂CH₂NH₂); 52.27 (N^{t}*C*H₂CH₂CH₂OH); 39.29 (-*C*H₂NH₂); 28.97 (CH₂*C*H₂CH₂)
MS (EI, m/z, rel. Int.):
176 (2.55, [M]+); 146 (100, M-H₂O]+); 102 (57.38); 58 (70.21)
IR (KBr Nujol, v, cm⁻¹):
3343 [s, br, V(NH₂) + v(OH)]; 2939, 2869 [s, v(C-H)]; 1593 [m, δ(NH₂)]; 1373 [m, δ(O-H)]; 1464 [s, δ(C-H)]; 1216 [m, v(C-N)); 1063 [s, v(C-O)]

### Beispiel 3: Typische Beispiele für Komplexe der allgemeinen Struktur [LₐM_{b}X_{c}]^{d} Y:

3.1.- einkernige Komplexe mit einem Liganden L
3.1.1. Ammin-(N(2-aminoethyI)N,N-bis(3-aminopropyI)amin]kupfer(II) bis(hexafluorophosphat), Cu(N₄3,3,2)NH₃(PF₆)₂
MS (FAB, positive Ionen, m/z, rel. Int.)
370 (15); 237 (100, [Cu(N₄3,3,2)]+; 216 (13); 178 (60)

| CHN-Analyse: C₈H₂₅N₅CuP₂F₁₂ M =544.79 g/mol | | | |
|---|---|---|---|
| Ber. | C 17.64 | H4.63 | N 12.86 |
| Gef | C 17.29 | H4.31 | N 12.52 |

3.1.2. Ammin[N,N-Bis(2-AminoethyI)N-(3-aminopropyI)amin] kupfer(II)-bis(hexafluorophosphat), Cu(N₄322)NH₃(PF₆)₂
MS (FAB, positive Ionen, m/z, rel. Int.)
446 (1); 368 (3); 223 (100, [Cu(N₄322)]+); 193 (17)

| CHN-Analyse: C ₇H₂₃N₅CuP₂F₁₂ M=530.77g/mol | | | |
|---|---|---|---|
| Ber. | C 15.84 | H4.37 | N 13.19 |
| Gef | C 15.74 | H4.21 | N 12.83 |

3.1.3. [2-Hydroxy-N,N-bis(2-aminoethyl)-ethylamin]-aqua-zink(II) bistetrafluoroborat [Zn(NN₂O2,2,2)(H₂O)](BF₄)₂
MS (FAB, positive Ionen, *m*/*z*, rel. Int.):
210 (7.67, [Zn(NN₂O2,2,2)]+); keine weiteren Fragmente

| CHN-Analyse: C₆H₁₈N₃O₂ZnBF₄ M=316.41 g/mol | | | |
|---|---|---|---|
| Ber. | C 22.78 | H 5.73 | N 13.28 |
| Gef | C 22.70 | H 5.81 | N 12.95 |

3.1.4. 322Ni(NO₃)₂
3.1.5. 332Ni(NO₃)₂
3.1.6. [322NiCl]₂Cl₂
3.1.7. [332NiCl(H₂O)]Cl
3.2. - zweikernige Komplexe mit einem Liganden L
3.2.1. Bis [N-(2-Aminoethyl)N,N-bis(3-aminopropyl)amin]dikupfer(II)tetranitrat
MS (FAB, positive Ionen, m/z, rel. Int.)
660 (1, M+-NO₃); 598 (1, M+-2NO₃); 536 (1, M+-3NO₃); 492 (2); 403 (2); 370 (14); 299 (32); 237 (100, [Cu(N₄3,3,2)]⁺)

| CHN-Analyse: C₁₆H₄₄N₁₂O₁₂Cu₂*H₂O M = 741.71 g/mol | | | |
|---|---|---|---|
| Ber. | C 25.91 | H 6.25 | N 22.66 |
| Gef | C26.46 | H 6.24 | N 22.97 |

3.2.2. Bis [N,N-Bis(aminoethyl)N-(3-aminopropyl)amin] di-kupfer(II)tetranitrat
MS (FAB, positive Ionen, m/z, rel. Int.)
632 (2, M+-NO₃); 570 (1, M+-2NO₃); 508 (0,5); 438 (1), 389 (2); 356 (1); 285 (54, [Cu(N₄322)NO₃]+); 223 (100 [Cu(N₄322)]+)

| CHN-Analyse: C₁₄H₄₀N₁₂O₁₂Cu₂ M = 695.64 g/mol | | | |
|---|---|---|---|
| Ber. | C24.17 | H 5.80 | N 24.16 |
| Gef | C 24.34 | H 5.71 | N 23.52 |

3.2.3. Di-µ²-dioxo{[3-Hydroxy-N-(2-aminoethyl)-N-(3-hydroxypropyl)-propylamin]-kupfer(II)} dihexafluorophosphat Methanol Disolvat, [(NNO₂2,3,3)Cu]₂(PF₆)₂ * 2 MeOH
MS (FAB, positive Ionen, *m/z,* rel. Int.):475 (18.95, [M-H]⁺; 301(37.67, [Cu(NNO₂2,3,3)+Cu]⁺; 239 (33.19, [Cu(NNO₂2,3,3)+H]⁺);

| CHN-Analyse: C₁₆H₃₈N₄O₄Cu₂P₂F₁₂ M = 767.52 g/mol | | | |
|---|---|---|---|
| Ber. | C 25.04 | H 4.99 | N 7.30 |
| Gef | C 25.09 | H 5.17 | N 7.46 |

IR (KBr, v, cm⁻¹):
3549, 3378, 3330 [w, v(OH) + v(NH₂)]; 2970, 2875, 2833 [m, v(C-H)]; 1596 [m, δ(NH2)]; 1473, 1459, 1433 [m, δ(C-H)]; 1065, 1046 [s, v(C-O)]; 833, 558 [s, v(PF₆)]
UV/VIS (CH₃OH, λ, nm, ε, 10^{3 .} cm^{2 .} mmol⁻³):
270(4.9); 366(2.0); 585 (0.12)
3.2.4. [(NOS₂322-2Me)Ni]₂
3.2.5. (NNO₂222)Zn(NNO₂222)Zn(OH)(BF₄)₂
3.3.- dreikernige Komplexe mit einem Liganden L
3.3.1. Tris [N,N-Bis(2-aminoethyl)N-(3-aminopropyl)amin-kupfer(11)] carbonattetrahexafluorophosphat
MS (FAB, positive Ionen, m/z, rel. Int.)
679 (1, [Cu₂(10)₂CO₃PF₆]+); 619 (1); 403 (2); 370 (22); 237 (100, Cu(10)+)

| CHN-Analyse: C₂₅H₆₆N₁₂O₃CuP₄F₂₄ M = 1353.37 g/mol | | | |
|---|---|---|---|
| Ber. | C22.18 | H 4.92 | N 12.42 |
| Gef | C 23.54 | H 6.22 | N 11.87 |

3.3.2. [(NNOS222)Ni]₂[(NNOS222)Ni]ClO₄
3.4.- vierkernige Komplexe mit einem Liganden L
Tetrakis-{µ2-Thio-[3-Hydroxy-N,N-bis[2-mercaptoethyl}propylamin] -zink(II)}, [Zn(NOS₂3,2,2)]₄
MS (FAB, positive Ionen, *m*/*z*, rel. Int.):
842 (4.45, [Zn₃(NOS₂3,2,2)₃]+Zn]⁺; 776 (3.23, [Zn₃(NOS₂3,2,2)₃]⁺; 581(26.18, [Zn₂(NOS₂3,2,2)+Zn]⁺; 519 (15.15, [Zn₂(NOS₂3,2,2)₂+H]⁺; 258 (100, [Zn(NOS₂3,2,2)+H]⁺)

| CHN-Analyse: C₂₈H₆₀N₄O₄S₈Zn₄ M= 1034.76∼mol | | | | |
|---|---|---|---|---|
| Ber. | C 32.50 | H 5.84 | N 5.41 | S 24.79 |
| Gef | C 32.94 | H 5.98 | N 5.67 | S 23.53 |

### Beispiel 4: Verwendung zum Bleichen

Die wässrige Bleichflotte enthält vorzugsweise die Metall-Komplexe, bezogen auf das Gewicht der Bleichflotte, in einer Menge von 0,001 bis 100 ppm Metall, insbesondere 0,01 bis 20 ppm Metall, vor allem 0,03 bis 10 ppm Metall (ppm bedeutet "parts per million", bezogen auf das Gewicht). Höhere Metall-Gehalte, etwa bis zu 500 ppm, können bei industriellen Bleichprozessen, beispielsweise auf dem Textil- oder Papiersektor, zweckmäßig sein. Die zuerst genannten niedrigen Metall-Gehalte beziehen sich vornehmlich auf Haushaltstextilwaschmittel.

Wässrige Wasch- und Bleichflotten, die Peroxyverbindungen und die erfindungsgemäßen Bleichkatalysatoren enthalten, sind im neutralen und alkalischen pH-Bereich, also von ca. pH 7 bis pH 14, wirksam. Ein Wirkungsoptimum liegt bei pH 8 bis pH 11.

### Beispiel 5: Verwendung in Wasch- und Reinigungsmittel-Formulierungen

Als wirksame Menge der Metall-Komplexe I sind hierbei üblicherweise Mengen von 0,0001 bis 0,5 Gew.-% Metall, insbesondere 0,00025 bis 0,25 Gew.-% Metall, vor allem 0,0005 bis 0,1 Gew.-% Metall, bezogen auf das Gewicht der Formulierungen, anzusehen. Diese Mengen können je nach landesüblichen Gepflogenheiten der Waschmittelzusammensetzungen leicht schwanken.

### Beispiel 6: Wirksamkeit der Bleich- und Oxidationskatalysatoren

Die erfindungsgemäßen Metallkomplexe eigenen sich in hervorragender Weise als Bleich und Oxidationskatalysatoren in Wasch- und Reinigungsmitteln und bei der Textil- und Papierbleiche. Ein wesentlicher Vorteil der erfindungsgemäßen Bleichkatalysatoren ist dabei die gute Löslichkeit und die Stabilität gegenüber Oxidation und Hydrolyse. Die Metallkomplexe selbst besitzen die katalytische Aktivität und sind in einer Vielzahl von Textilwaschmittelformulierungen hochwirksam. Sie verbessern in solchen Formulierungen die Bleichwirkung von Wasserstoffperoxid sowie von organischen und anorganischen Peroxysäure-Verbindungen. Weitere anwendungstechnische Vorteile sind die universelle Wirksamkeit bei der Entfernung hydrophiler wie auch hydrophober Verschmutzungen sowie ihre Verträglichkeit mit üblichen Wachmittel-Enzymen wie Proteasen, Cellulasen, Lipasen, Amylasen und Oxidasen.

Die erfindungsgemäßen Bleichkatalysatoren wurden bezüglich ihrer Bleichwirkung mit dem Morin-Test untersucht. Dabei wurden beispielsweise für den Komplex [Cu(N₄-332)(NH₃)]PF₆ folgende Werte gefunden:

| | % Entfärbung im Morin-Test | |
|---|---|---|
| Zeit [min] | ohne TAED | mit 3% TAED (Tetraacetylethylendiamin) |
| 5 | 13 | 19 |
| 10 | 16 | 36 |
| 15 | 17 | 55 |
| 20 | 19 | 76 |
| 25 | 21 | 91 |
| 30 | 22 | 94 |

[Cu(N₄-332)(NH₃)]PF₆ entspricht

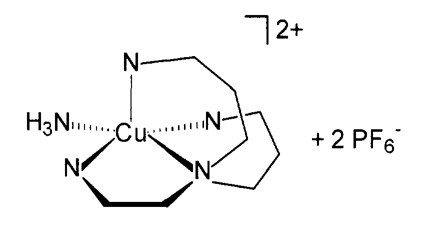

Messbedingungen für Morin-Test: 1 g/l PBMH, 20°C, pH = 9.5, 0.25 mg/l Cu

## Patentansprüche

1. Bleich- und Oxidationsmittel, die Metallkomplexe mit einem oder mehreren unsymmetrisch substituierten tripodalen Liganden enthalten, **dadurch gekennzeichnet, dass** sich die Substituenten am zentralen Ligandenatom des Tripods durch die Länge der Alkylengruppen zwischen dem zentralen Ligandenatom und den direkt koordinierenden Donoratomen und/oder durch unterschiedliche Donoratome unterscheiden.

2. Bleich- und Oxidationsmittel nach Anspruch 1, **dadurch gekennzeichnet, dass** die Substituenten am zentralen Ligandenatom des Tripods
a) mindestens zwei unterschiedliche Alkylengruppen, aber gleiche Donorgruppen besitzen oder
b) gleiche Alkylengruppen, aber Donorgruppen mit mindestens zwei unterschiedlichen Donoratomen besitzen
oder
c) mindestens zwei unterschiedliche Alkylengruppen und unterschiedliche Donorgruppen besitzen.

3. Bleich- und Oxidationsmittel nach Anspruch 1 und 2, **dadurch gekennzeichnet, dass** die tripodalen Liganden
a) die allgemeinen Formel I 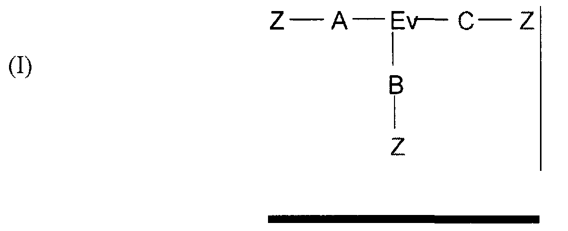 besitzen, in der Eᵥ für ein Element der 5. Hauptgruppe, A, B und C eine C₂- bis C₄-Alkylengruppe bezeichnen mit A ≠ B oder A ≠ B ≠ C; Z, für -NR¹R², -SR³ und -OR⁴ steht, wobei R¹ bis R⁴ für Wasserstoff, Alkyl, Aryl und substituiertem Aryl stehen
oder
b) die allgemeine Formel II 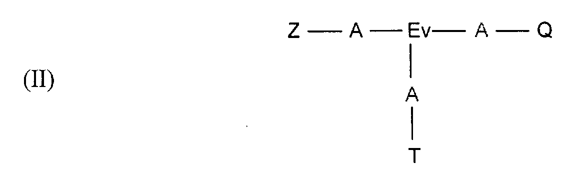 besitzen, in der Eᵥ für ein Element der 5. Hauptgruppe, A eine C₂- bis C₄-Alkylengruppe bezeichnet, wobei Z, Q und T für -NR¹R², -SR³ und -OR⁴ stehen, mit Z ≠ Q oder Z ≠ Q ≠ T, wobei R¹ bis R⁴ für Wasserstoff, Alkyl, Aryl und substituiertem Aryl stehen und sich Z, Q und T mindestens in einem Donoratom unterscheiden, wobei R¹ bis R² für Wasserstoff, Aryl und substituiertes Aryl, insbesondere mono oder bis Amino-, Hydroxy- und Mercaptoaryl stehen
oder
c) die allgemeine Formel III 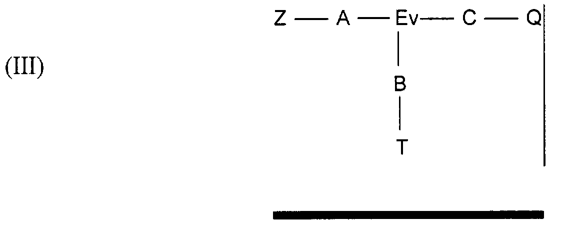 besitzen, in der Eᵥ für ein Element der 5. Hauptgruppe, A, B und C eine C₂- bis C₄-Alkylengruppe bezeichnen mit A ≠ B oder A ≠ B ≠ C, Z, Q und T unabhängig voneinander mit Z ≠ Q oder Z ≠ Q ≠ T, wobei Z, Q und T für -NR¹R², -SR³ und -OR⁴ stehen, wobei R¹ bis R⁴ für Wasserstoff, Alkyl, Aryl und substituiertem Aryl stehen und sich Z, Q und T mindestens in einem Donoratom unterscheiden.

4. Bleich- und Oxidationsmittel nach Anspruch 1 bis 3, **dadurch gekennzeichnet, dass** die Metall-Komplexe ein- oder mehrkernige Komplexe sind.

5. Bleich- und Oxidationsmittel nach Anspruch 1 bis 4, **dadurch gekennzeichnet, dass** die Metall-Komplexe die allgemeine Formel I (I) [LₐM_{b}X_{c}]^{d} Y
besitzen, wobei M für ein Übergangsmetall Oxidationsstufe I, II, III, IV, V und/oder VI steht, X eine Koordinations- oder Brückengruppe darstellt, Y ein Gegenion in der entsprechenden stöchiometrischen Menge zum Ausgleich einer vorhandenen Ladung d bezeichnet, wobei d als Ladung des Metall-Komplexes positiv, null oder negativ sein kann, a und b unabhängig voneinander ganze Zahlen von 1 bis 8 bedeuten und c eine ganze Zahl von 0 bis 32 bezeichnet.

6. Bleich- und Oxidationsmittel nach Anspruch 1 bis 5, **dadurch gekennzeichnet, dass** als Koordinations- oder Brückengruppe X Cl⁻, Br⁻, I⁻, F⁻, SCN⁻, I₃⁻, OH⁻, O₂²⁻, O²⁻, O₂⁻, OOH⁻, H₂O, SH⁻, CN⁻, OCN⁻, HCO₃⁻, CO₃²⁻, S₄²⁻, S²⁻, SO, N₃⁻, N³⁻, NH₃, N(R⁵)₃, N(R⁵)₂⁻, R⁵O⁻, R⁵COO⁻, (R⁵)₃C-CO-CH-C(O⁻)-(R⁵)₃, R⁵SO₃⁻ oder R⁵SO₄- fungiert, wobei R⁵ jeweils Wasserstoff, C1-bis C8-Alkyl, C7- bis C15-Aralkyl oder C6- bis C18-Aryl bezeichnen.

7. Bleich- und Oxidationsmittel nach Anspruch 1 bis 6, **dadurch gekennzeichnet, dass** das Gegenion Y
a) - bei positivem d: Cl⁻, Br⁻, I⁻, F⁻, NO₃⁻, ClO₄⁻, SCN⁻, PF₆⁻, R⁵SO₄⁻, R⁵COO⁻, CF₃COO⁻, R⁵SO₃⁻, BF₄⁻, B(Ph)₄⁻, SO₄²⁻ oder SO₃²⁻ oder
b) - bei negativem d: Li⁺, Na⁺, K⁺, Mg²⁺, Ca²⁺, Sr²⁺, Ba²⁺, NH₄⁺, R⁵NH₃⁺, (R⁵)₂NH₂⁺, (R⁵)₃NH⁺ und (R⁵)₄N⁺ ist.

8. Bleich- und Oxidationsmittel nach Anspruch 1 bis 4, **dadurch gekennzeichnet, dass** es sich um Metallkomplexe mit einem oder mehreren der folgenden Liganden handelt:
a) N-(2-Aminoethyl)N,N-bis(3-aminopropyl)amin, H₂NCH₂CH₂N(CH₂CH₂CH₂NH₂)₂
b) N,N-Bis(aminoethyl)N-(3-aminopropyl)amin, (H₂NCH₂CH₂)₂NCH₂CH₂CH₂NH₂
c) 2-Hydroxy-N,N-bis(2-aminoethyl)-ethylamin, HOCH₂CH₂N(CH₂CH₂NH₂)₂
d) 2-Amino-N,N-bis(2-hydroxyethyl)-ethylamin, H₂NCH₂CH₂N(CH₂CH₂OH)₂ oder
e) 3-Hydroxy-N-(2-aminoethyl)-N-(3-hydroxypropyl)-amin, HOCH₂CH₂CH₂N(CH₂CH₂CH₂OH)(CH₂CH₂NH₂).

9. Bleich- und Oxidationsmittel nach Anspruch 1 bis 5 und 7, **dadurch gekennzeichnet, dass** es sich um folgende Metallkomplexe handelt:
a) Ammin-(N(2-aminoethyI)N,N-bis(3-aminopropyI)amin]kupfer(II) bis(hexafluorophosphat), Cu(N₄3,3,2)NH₃(PF₆)₂ oder
b) Ammin[N,N-Bis(2-AminoethyI)N-(3-aminopropyI)amin] kupfer(II)-bis(hexafluorophosphat), Cu(N₄3,2,2)NH₃(PF₆)₂ oder
c) [2-Hydroxy-N,N-bis(2-aminoethyl)-ethylamin]-aqua-zink(II) bistetrafluoroborat [Zn(NN₂O2,2,2)(H₂O)](BF₄)₂ oder
d) 322Ni(NO₃)₂ oder
e) 332Ni(NO₃)₂ oder
f) [322NiCl]₂Cl₂ oder
g) [332NiCl(H₂O)]Cl.

10. Bleich- und Oxidationsmittel nach Anspruch 1 bis 7, **dadurch gekennzeichnet, dass** es sich um folgende Metallkomplexe handelt:
a) Bis [N-(2-Aminoethyl)N,N-bis(3-aminopropyl)amin]dikupfer(II)tetranitrat oder
b) Bis [N,N-Bis(aminoethyl)N-(3-aminopropyl)amin] di-kupfer(II)tetranitrat oder
c) Tris [N,N-Bis(2-aminoethyl)N-(3-aminopropyl)amin-kupfer(II)] carbonattetrahexafluorophosphat oder
d) Tetrakis-{µ2-Thio- [3-Hydroxy-N,N-bis[2-mercaptoethyl}propylamin]-zink(II)}, [Zn(NOS₂3,2,2)]₄
e) Di-µ²-dioxo{[3-Hydroxy-N-(2-aminoethyl)-N-(3-hydroxypropyl)-propylamin]-kupfer(II)} dihexafluorophosphat Methanol Disolvat, [(NNO₂2,3,3)Cu]₂(PF₆)₂ * 2 MeOH
f) [(NOS₂322-2Me)Ni]₂
g) (NNO₂222)Zn(NNO₂222)Zn(OH)(BF₄)₂
h) [(NNOS222)Ni]₂[(NNOS222)Ni]ClO₄.

11. Verwendung von Metallkomplexen gemäß Anspruch 1 als Bleich- und Oxidationskatalysatoren.

12. Verwendung nach Anspruch 11
a) in Wasch- und Reinigungsmitteln oder
b) in Wasch- und Reinigungsmittel-Formulierungen oder
c) zum Bleichen von Textilien oder
d) zum Bleichen von Papier oder
e) zum Bleichen von verschmutzten Substraten oder
f) zum Färben von Haaren oder
g) zum Bleichen von Haaren oder
h) zur Aktivierung von Luftsauerstoff.
